# EUROPEAN PATENT SPECIFICATION

(11) **EP 2 948 082 B1**
(45) Date of publication and mention of the grant of the patent: **07.04.2021**
(21) Application number: 14706368.9
(22) Date of filing: 28.01.2014
(51) Int. Cl.: A61B 17/86, A61B 17/74

(54) **LIMITED COLLAPSE SURGICAL SCREWS**
BEGRENZT FALTBARE CHIRURGISCHE SCHRAUBEN
VIS CHIRURGICALES À PLIAGE LIMITÉ

(43) Date of publication of application: 02.12.2015
(73) Proprietor: Biomet Manufacturing, LLC, Warsaw, IN 46581 (US)
(72) Inventor: METZINGER, Anthony, Warsaw, IN 46580 (US); SLAGLE, Paul, Leesburg, IN 46538 (US)
(74) Representative: Mays, Julie
(86) International application number: PCT/US2014/013334
(87) International publication number: WO 2014/117132

(56) References cited:
- WO-A1-2012/158351
- US-A1- 2010 312 245

## Description

### CROSS-REFERENCE TO RELATED APPLICATIONS

### FIELD OF THE INVENTION

The present invention is directed to orthopedic devices and, more specifically, to telescopic orthopedic screws including limited collapse sleeves and associated bushings. Extendable bone screws comprising first and second screw sections are known, for example, from US 2010/312245 and WO 2012/158351.

### INTRODUCTION TO THE INVENTION

The present invention is defined in the independent claims, to which reference should now be made. Advantageous embodiments are set out in the sub claims.

### BRIEF DESCRIPTION OF THE DRAWINGS

FIG. 1 is an elevated perspective view of a first exemplary telescoping surgical screw.
FIG. 2 is an elevated perspective view of the distal shaft comprising a part of the first exemplary telescoping surgical screw of FIG. 1.
FIG. 3 is a cross-sectional view of the first exemplary telescoping surgical screw of FIG. 1.
FIG. 4 is an elevated perspective view of the proximal sleeve comprising a part of the first exemplary telescoping surgical screw of FIG. 1.
FIG. 5 is an elevated perspective view of the distal shaft comprising a part of the first exemplary telescoping surgical screw of FIG. 1.
FIG. 6 is an elevated perspective view of the reconfigurable bushing comprising a part of the first exemplary telescoping surgical screw of FIG. 1.
FIG. 7 is another elevated perspective view of the reconfigurable bushing comprising a part of the first exemplary telescoping surgical screw of FIG. 1.
FIG. 8 is a cross-sectional view of the reconfigurable bushing of FIGS. 6 and 7.
FIG. 9 is a profile view of the cross-section of the reconfigurable bushing of FIG. 8.
FIG. 10 is an elevated perspective view of a distal end of an exemplary inserter for use with the reconfigurable bushing of FIG. 6.
FIG. 11 is an elevated perspective view of a distal end of an exemplary inserter mounted to the reconfigurable bushing of FIG. 6.
FIG. 12 is a cross-sectional view of the first exemplary telescoping surgical screw of FIG. 1 and a cross-sectional view of the exemplary inserter of FIG. 10.
FIG. 13 is a magnification of the cross-sectional view of the first exemplary telescoping surgical screw of FIG. 1 and the cross-sectional view of the distal end of the exemplary inserter of FIG. 10.
FIG. 14 is an elevated perspective view of a second exemplary reconfigurable bushing shown in a partially rolled shape.
FIG. 15 is an elevated perspective view of the second exemplary reconfigurable bushing of FIG. 14 shown further rolled up.
FIG. 16 is an elevated perspective view of a third exemplary reconfigurable bushing.
FIG. 17 is a profile view of the third exemplary reconfigurable bushing of FIG. 16.
FIG. 18 is an elevated perspective view of the third exemplary reconfigurable bushing of FIG. 16.
FIG. 19 is an elevated perspective view of an exemplary positioning screw.
FIG. 20 is an elevated perspective view of the exemplary positioning screw of FIG. 19.
FIG. 21 is a cross-sectional view showing preliminary engagement between the exemplary positioning screw of FIG. 19 and the third reconfigurable bushing of FIG. 16.
FIG. 22 is a cross-sectional view showing final engagement between the exemplary positioning screw of FIG. 19 and the third reconfigurable bushing of FIG. 16.
FIG. 23 is an elevated perspective view of a fourth exemplary reconfigurable bushing.
FIG. 24 is an elevated perspective view of the fourth exemplary reconfigurable bushing of FIG. 23.
FIG. 25 is a cross-sectional view showing engagement between the exemplary positioning screw of FIG. 19 and the fourth reconfigurable bushing of FIG. 23.

### DETAILED DESCRIPTION

The exemplary embodiments of the present disclosure are described and illustrated below to encompass orthopedic devices and processes and, more specifically, to telescopic orthopedic screws including limited collapse sleeves and associated bushings. However, for clarity and precision, the exemplary embodiments as discussed below may include optional steps, methods, and features that one of ordinary skill should recognize as not being a requisite to fall within the scope of the present disclosure.

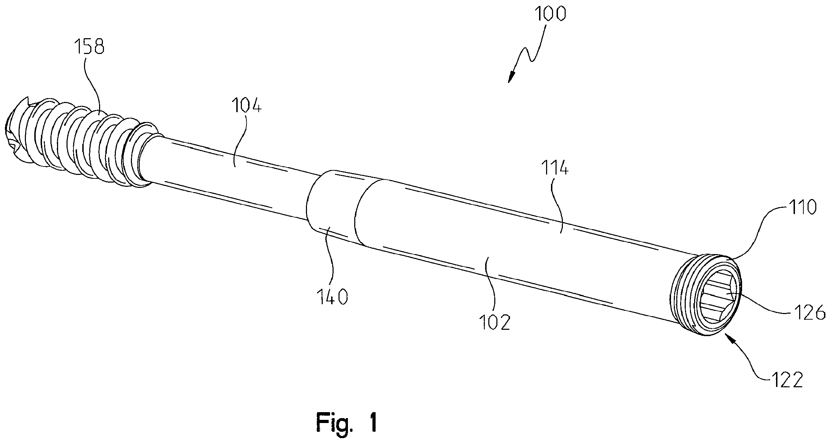

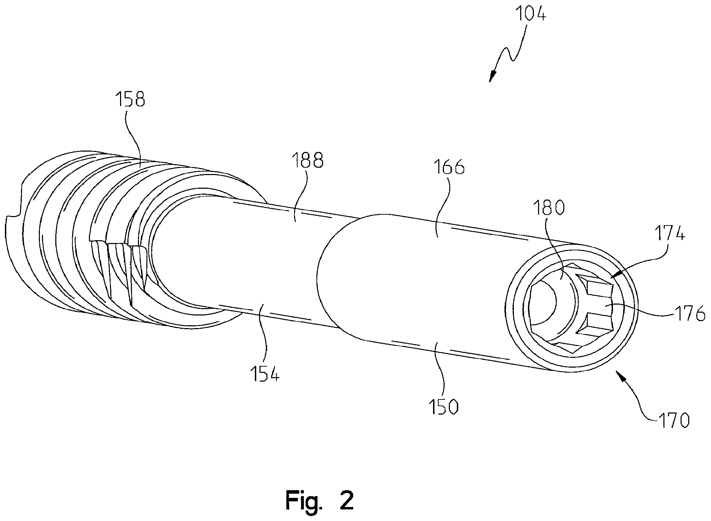

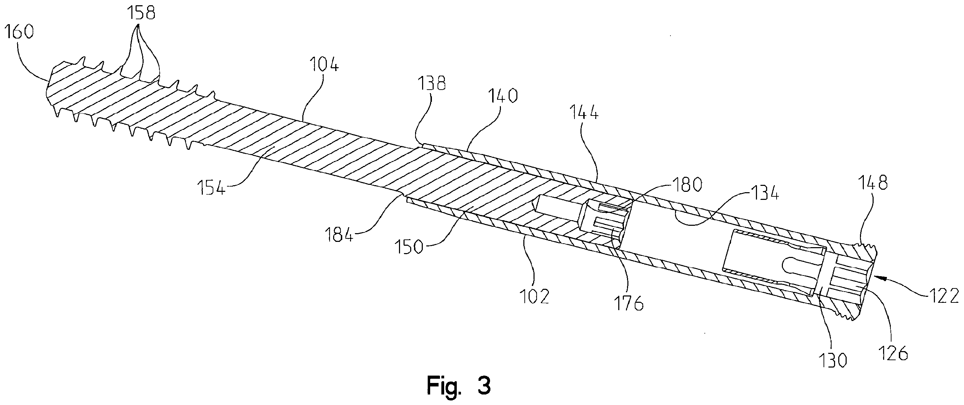

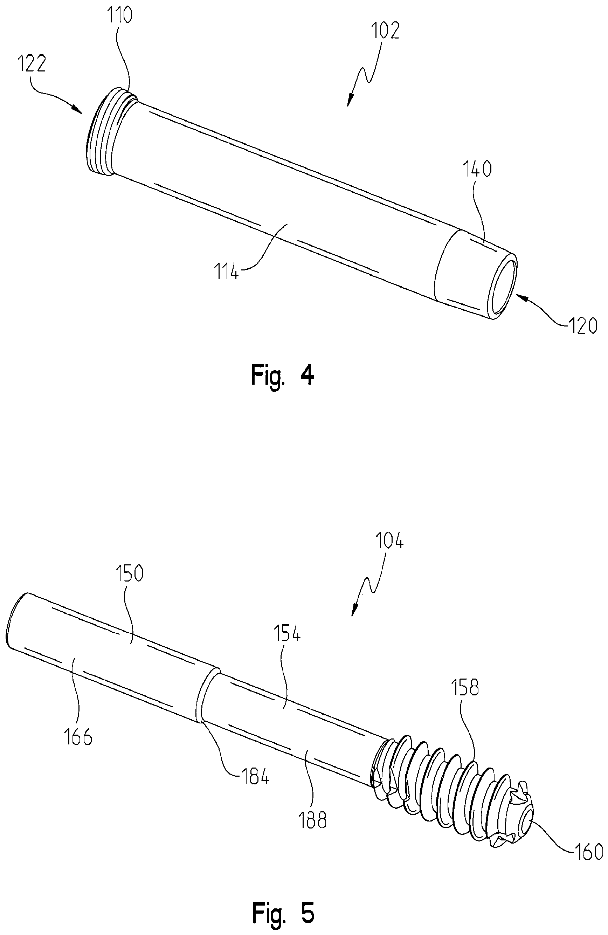

Referencing FIGS. 1-5, a first exemplary telescoping screw 100 includes a proximal sleeve 102 and a distal shaft 104 that are longitudinally repositionable with respect to one another to change the overall longitudinal length of the screw.

In exemplary form, the proximal sleeve 102 includes a proximal threaded head 110 that is integral with a distally extending cylindrical housing 114. Both the threaded head 110 and the cylindrical housing 114 are hollow representative of a through hole that extends between a distal orifice 120 and a proximal orifice 122. More specifically, the proximal orifice 122 is hexagonal and bounded by six planar walls 126 that extend distally to a flange 130 that changes the cross-section from hexagonal to circular. Where the planar walls 126 intersect the flange 130, the diameter of the opening extending through the flange is the same as the distance between opposed planar walls.

Extending distally, the underside of the flange 130 intersects an inner circumferential wall 134 of the cylindrical housing 114, which operates to increase the diameter of the through opening. This change in cross-sectional area inhibits the proximal portion of the distal shaft 104 from passing through the circular opening of the flange 130. Consequently, the screw 100 has its shortest length when the proximal portion of the distal shaft 104 abuts the underside of the flange 130. The inner circumferential wall 134 has a constant diameter until reaching the distal orifice 120. The distal orifice 120 is circular in cross-section, but has a diameter that is less than that of the inner circumferential wall 134. The distal orifice 120 is delineated by an inwardly protruding lip 138 that is operative to retain a proximal portion of the shaft 104 within the housing 114.

An exterior of the cylindrical housing 114 includes a tapered surface 140 having a circular longitudinal profile that decreases in outside diameter slightly from proximal to distal until reaching the protruding lip 138. Seamlessly adjoining the tapered surface 140 is primary outer surface 144 having a circular longitudinal profile and a constant diameter. The outer surface 144 extends proximally until reaching threads 148 that circumscribe the proximal head 110. In this exemplary embodiment, the threads 140 extending circumferentially to increase the diameter of the head 110 in excess of the diameter of the primary outer surface 144.

Referring to FIGS. 1-3 and 5, the distal shaft 104 includes a proximal section 150 that is integrally formed with a column 154 having a series of helical threads 158 that extend longitudinally until reaching a distal tip 160. The proximal section 150 is cylindrical in shape and includes an exterior surface 166 having a substantially circular cross-section with a constant diameter. A proximal end 170 of the shaft 104 includes a hexagonal orifice 174 bounded by six planar walls 176 that extend distally to a flange 180 that changes a cross-section of the cavity from hexagonal to circular. Where the planar walls 176 intersect the flange 180, the diameter of the opening extending through the flange is the same as the distance between opposed planar walls. The circular cross-section of the flange 180 is maintained for a predetermined longitudinal distance, after which the cross-section tapers distally to define a conical end cavity that leads into a central bore cavity 182 longitudinally extending through to a distal opening at the distal tip 160; otherwise, the proximal section 150 comprises solid material.

Extending distally from the proximal section 150 is the column 154, with a tapered section 184 therebetween. This tapered section 184 provides a transition from the larger external diameter of the proximal section 150 to the slightly smaller external diameter of a smooth portion 188 of the column. This smooth portion 188 has a cylindrical shape with a substantially constant external diameter until reaching the helical threads 158. Upon reaching the helical threads 158, the outside diameter increases and is larger than the outside diameter of the proximal section 150.

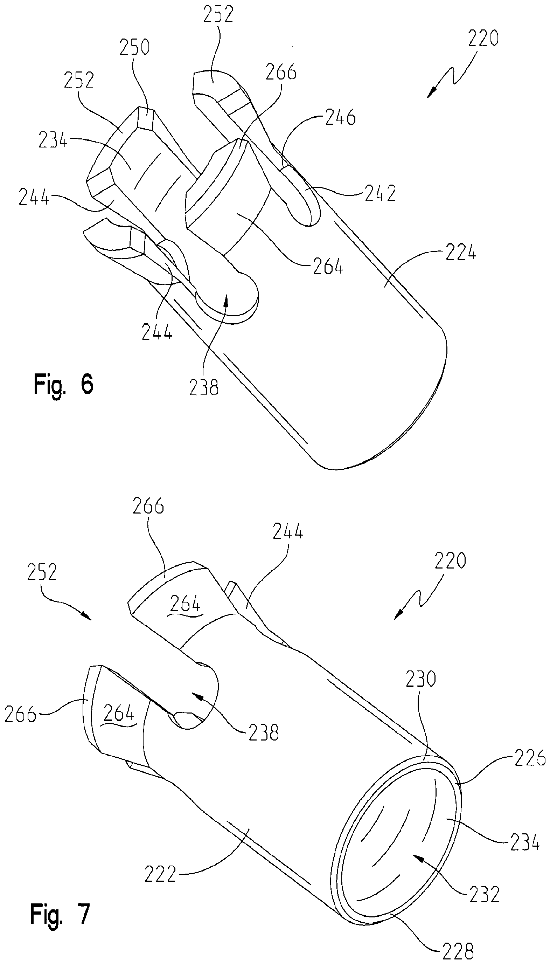

Referring to FIGS. 6-9, a first exemplary reconfigurable bushing 220 may comprise a part of the first exemplary telescoping screw 100. The bushing 220 may be fabricated from any number of various materials that include, without limitation, a polymer, a metal, a metal alloy, a composite, or any other material that lends itself to reshaping and has at least a predetermined elasticity. By way of example, the reconfigurable bushing 220 comprises a metal alloy. In exemplary form, the first reconfigurable bushing 220 includes a circumferential wall 222 having a circular outer circumferential surface 224 extending along a majority of the longitudinal length thereof. A distal end 226 of the bushing 220 is flat and delineated by a ring-shaped end surface 228 perpendicular to the outer circumferential surface 224. In exemplary form, the ring-shaped end surface 228 and the outer circumferential surface 224 are circumferentially interposed by a tapered ring surface 230. The ring-shaped end surface 228 has a generally uniform radial thickness and includes a through opening 232 that extends longitudinally along the bushing. An inner circumferential surface 234 provides a cylindrical boundary for the distal through opening 232 that is substantially uniform until reaching a series of cut-outs 238.

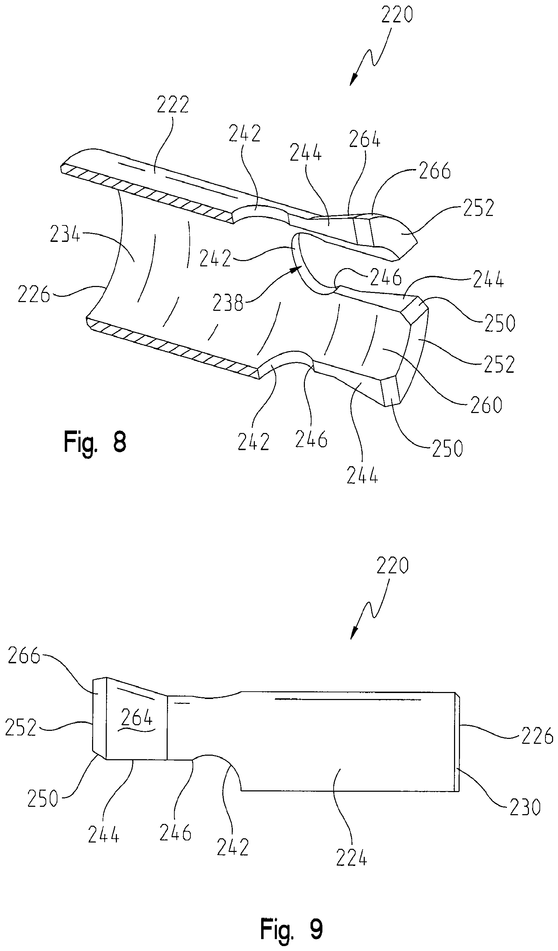

Each cut-out 238 extends radially through the bushing 220 and the center of which is equidistantly spaced from an adjacent cut-out. In exemplary form, the bushing 220 includes four cut-outs 238 having centers that are circumferentially off-set from one another ninety degrees. It should be noted, however, that fewer than four cut-outs 238 may be utilized or greater than four cut-outs may be utilized. By way of example, each cut-out 238 includes a distal circular profile delineated by an arcuate edge 242 that intersects a pair of parallel edges 244 partially delineating a rectangular profile. In this exemplary embodiment, the diameter of the arcuate edge 242 is greater than the distance between the parallel edges 244 to create a lip 246 operative to retain a corresponding feature of an inserter (see FIG. 8). Proximate the proximal end of the parallel edges 244, the cut-outs 238 taper via tapered edges 250 to increase a circumferential spacing of the cut-outs until reaching a proximal end 252 of the bushing 220, which is adjacent the most proximal portion of the tapered edges.

In exemplary form, the orientation and repetition of the cut-outs 238 is operative to create a number of circumferentially equidistant fingers 260 that extend away from the distal end 226 to delineate a proximal end 252. Approximately half way longitudinally along the fingers 260, the circumferential wall 222 begins to gradually increase in radial length embodied in a frustroconical exterior surface 264. This gradual circumferential wall 222 thickness increase is exhibited in the radial thickness of the fingers 260 increasing in the longitudinal direction. But this increase in outer diameter of the circumferential wall is in contrast to the longitudinal diameter of the inner circumferential surface 234, which remains substantially constant but for the cut-outs.

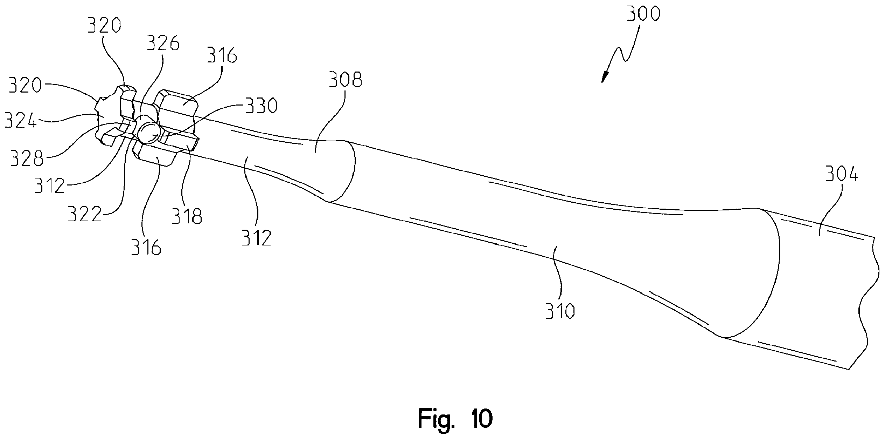

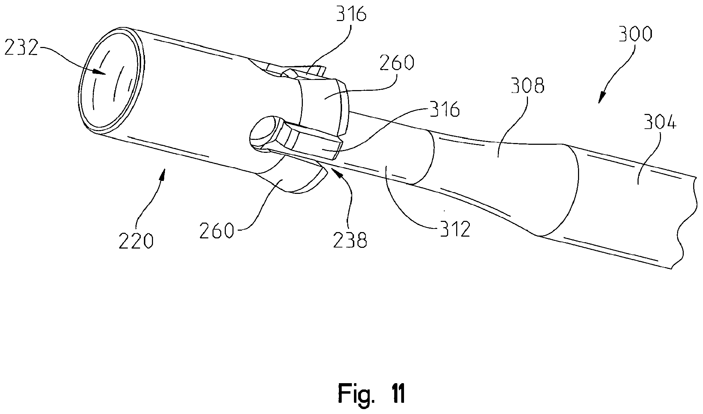

As shown in FIGS. 10 and 11, an exemplary inserter 300, which does not form part of the claimed invention, may be used to selectively engage and move the first exemplary reconfigurable bushing 220 from outside the proximal sleeve 102 to seat against the inner circumferential wall 134. In exemplary form, the inserter 300 includes an elongated shaft 304 that includes a frustroconical outer surface 308 interposing a first circumferential surface 310 and a second circumferential surface 312. The outside diameter of the second circumferential surface 312 is less than the outside diameter of the first circumferential surface 310.

Along the length of the second circumferential surface 312 are four generally rectangular projections 316 that are uniformly circumferentially distributed. Each projection 316 is oriented radially perpendicular to the second circumferential surface 312 and includes a block rectangular shape with two tapered surfaces. The height (radial length) of the projections 316 and the diameter of the second circumferential surface 312 are selected in order to ensure that the projections extend into and are received by the cutouts 238 of the reconfigurable bushing 220. In other words, the circumferentially equidistant fingers 260 interpose the projections 316. Extending distally from the projections 316, the second circumferential surface 312 continues its cylindrical exterior until reaching a second set of projections 320 and a pair of pins 322 interposing the first and second sets of projections 316, 320.

The pins 322 extend radially outward from the second circumferential surface 312 in opposite directions and are longitudinally aligned with two of the first and second sets of projections 316, 320. In exemplary form, each projection includes a cylindrical shape having an outer circumferential surface 326 that terminates at a planar circular surface 330 that is perpendicular to the outer circumferential surface. As will be discussed in more detail hereafter, each of the pins 322 is adapted to be received within the cut-outs 238 of the bushing 220 to mount the inserter 300 to the bushing.

The second set of projections 320 comprise four rectangular blocks that are uniformly circumferentially distributed around the second circumferential surface. Each projection 320 is oriented radially perpendicular to the second circumferential surface 312 and includes a block rectangular shape with a single tapered surface. The height (radial length) of the projections 320 and the diameter of the second circumferential surface 312 are selected in order to ensure that opposing projections have an outside dimension (i.e., outside diameter) that is less than the inside diameter of the inner circumferential surface 234 of the bushing 220. Extending distally from the projections 320, the inserter 300 includes a substantially flat distal surface 324.

Referring back to FIGS. 1-13, assembly of first exemplary telescoping screw 100 takes place prior to the forming the protruding lip 138 of the proximal sleeve 102. In exemplary form, the proximal portion 150 of the distal shaft 104 is inserted through the distal opening 120 of the proximal sleeve 102. It is presumed that the reconfigurable bushing 220 is not located within the proximal sleeve 102 and seated against the inner circumferential wall 134 prior to insertion of the distal shaft 104 into the proximal sleeve 102. The proximal portion 150 of the distal shaft 104 is inserted into the proximal sleeve at a depth sufficient for the tapered section 184 to pass through the distal opening 120. After the tapered section 184 has passed through the distal opening 120, a tool (not shown) is used to circumferentially crimp the distal end of the tapered surface 140 to create the protruding lip 138. After the protruding lip 138 is formed, the tapered section 184 has a larger diameter than the diameter of the opening delineated by the protruding lip, thereby inhibiting the proximal portion 150 of the distal shaft 104 from being removed from the interior of the proximal sleeve 102. But at the same time, the longitudinal length of the proximal portion 150 of the distal shaft 104 is less than the longitudinal length between the protruding lip 138 and the flange 130, thus allowing for movement of the sleeve 102 with respect to the shaft 104. More specifically, a maximum longitudinal length of the first exemplary telescopic screw 100 is reached when the tapered section 184 abuts the protruding lip 138. Conversely, the minimum longitudinal length of the first exemplary telescopic screw 100 is reached when the proximal end 170 abuts the flange 130.

In order to constrain the longitudinal length variance of the first exemplary telescoping screw 100, a bushing may be inserted into the proximal sleeve 102 to occupy a portion of the internal cavity, thereby limiting the longitudinal travel of the shaft 104 with respect to the sleeve 102. More specifically, the first reconfigurable bushing 220 may be inserted into the proximal sleeve 102 after the proximal sleeve 102 and distal shaft 104 have been mounted to one another.

In exemplary form, the reconfigurable bushing 220 is first mounted to the exemplary inserter 300. To do so, the distal surface 324 and the second set of projections 320 are inserted into the proximal end 252 of the bushing 220. In this exemplary embodiment, the second set of projections 320 are circumferentially aligned and equidistantly spaced apart from one another, as well as each having the same radial height. Accordingly, the outer circumferential surface 328 of each projection 320 has an arc of a circle having a diameter slightly smaller than the diameter of the inner circumferential surface 234 of the bushing 220. In this manner, when the second set of projections 320 are inserted through the proximal end 252 of the bushing 220, continued distal movement of the inserter 300 with respect to the bushing causes the inserter to penetrate further into the interior of the bushing where a friction fit is formed between the projections and the inner circumferential surface 324. At the same time, the pins 322 and the first set of projections 316 are aligned with the cut-outs 238 of the bushing 220 so that each pin and projection is received within a corresponding one of the cut-outs. In particular, the diameter of each pin 322 is slightly larger than the distance between opposed parallel edges 244 partially delineating a cut-out 238 so that insertion of the pin into a cut-out forces the adjacent fingers 260 away from one another until the pin reaches the circular profile delineated by the arcuate edge 242. Upon reaching the arcuate edge 242, each pin 322 clears the parallel edges 244 and is secured in the cut-out by the tapered edges 250 retarding egress of the pin away from the arcuate edge. Nevertheless, the pins 322 may disengage from the bushing 220 by applying sufficient force to overcome the force maintaining the fingers 260 in position and cause the pins to pass beyond the arcuate edges 242.

In this exemplary embodiment, the radial height of each projection 316 is longer than the projections 320 of the second set. Accordingly, the outer circumferential surface 318 of each projection 316 has an arc of a circle having a diameter slightly smaller than the outside diameter of the outer circumferential wall 222 of the bushing 220. As a result, when the distal end of the inserter 300 is inserted into the proximal end of the bushing 220, and the projections 316 and pins 322 are aligned with the cut-outs 238, the diameter of the pins 322 inhibits the pins from passing beyond the arcuate edge 242, thereby inhibiting further penetration of the inserter into the bushing. But because of the dimensions of the pins 322 and projections 316, the pins and projections extend through a corresponding cut-out 238 so that rotation of the inserter 300 will necessarily result in rotation of the bushing 220. It should also be noted that an outside diameter of the first and second set of projections 316, 320, measured from the outer circumferential surfaces 318, 328 of opposing projections, is less an inner diameter of the flange 130 of the proximal sleeve 102 in order to allow the projections 316, 320 to pass through the flange in either direction without obstruction. Likewise, the outside diameter of the pins 322, measured from the planar circular surfaces 330 of the pins, is less an inner diameter of the flange 130 of the proximal sleeve 102 in order to allow the pins to pass through the flange in either direction without obstruction.

After the bushing 220 has been mounted to the inserter 300, the inserter may be used to position the bushing within the interior of the proximal sleeve 102. In exemplary form, the inserter 300 and bushing 220 are oriented so that the distal end 226 of the bushing is longitudinally aligned with the proximal orifice 122 of the proximal sleeve 102. The bushing 220 and inserter 300 are repositioned with respect to the proximal sleeve 102 so that the bushing and inserter pass through the proximal orifice 122 and through the circular opening delineated by the flange 130. It should be noted that the outer circumferential surface 224 at the distal end 226 of the bushing 220 has a diameter that is slightly less than that of the interior diameter of the circular opening delineated by the flange 130, thereby allowing the distal end to pass through the flange unimpeded. But the same circumstance is not present with respect to the proximal end 252.

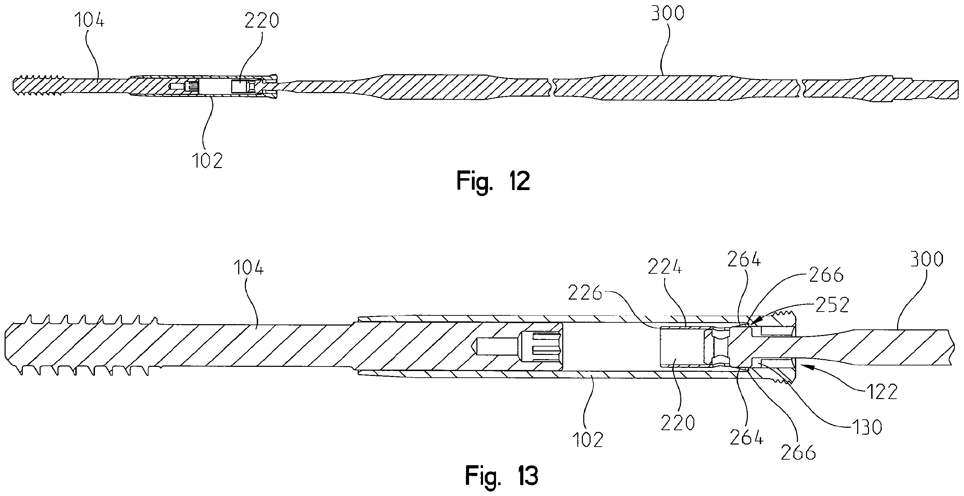

When the frustroconical exterior surfaces 264 of the fingers 260 reach the flange 130, the external diameter of the frustroconical exterior surfaces is larger than the circular opening delineated by the flange. Accordingly, the longitudinal force of the inserter 300 continuing to move the bushing 220 distally with respect to the proximal sleeve 102 causes the fingers 260 to flex or deform elastically inward (toward the longitudinal axis of the bushing) to allow the fingers to pass through the circular opening of the flange. But after the proximal end 252 of the bushing 220 passes distally beyond the flange 130, the fingers 260 flex or deform elastically outward to take on their original dimensions. At the time the fingers 260 take on their original shape, the diameter at the tapered circumferential surfaces 266 of opposing fingers is greater than the diameter of the circular opening delineated by the flange 130, thereby prohibiting the bushing 220 from proximally passing through the flange. After the fingers 260 have completely passed distally beyond the flange 130, the inserter 300 may be disengaged and proximally withdrawn through the flange and through the proximal orifice 122 of the proximal sleeve 102. As can be seen in FIG. 13, the maximum travel of the distal shaft 104 with respect to the proximal sleeve 102 has been reduced as a result of the insertion of the bushing 220. Moreover, the through opening 232 of the bushing 220 allows for through put of a driver (not shown) that is received within the hexagonal orifice 174 of the distal shaft 104 in order to rotate the distal shaft with respect to the proximal sleeve 102 even after the bushing is inserted into the proximal sleeve. It should also be understood, that the driver may engage the hexagonal orifice 174 of the distal shaft 104 in order to rotate the distal shaft with respect to the proximal sleeve 102 prior to insertion of the bushing 220 into the proximal sleeve.

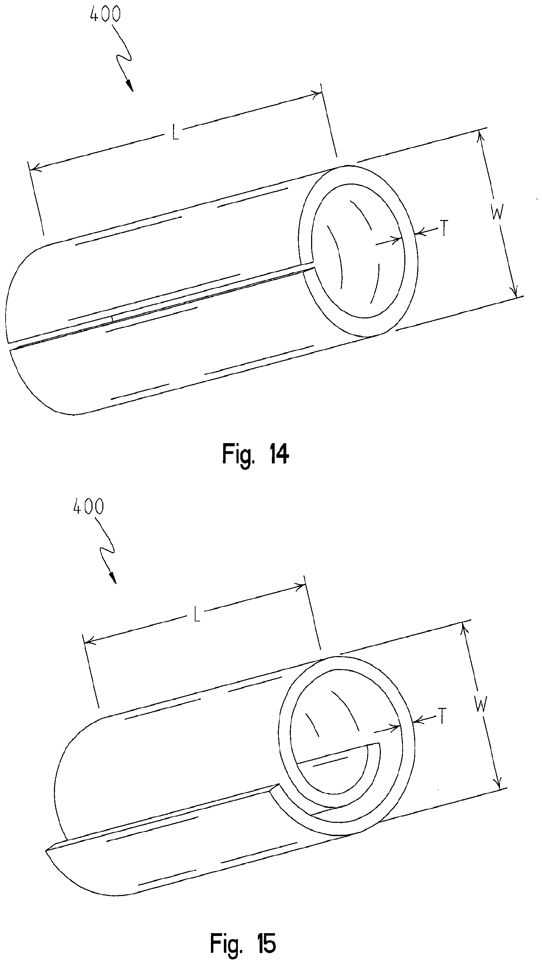

Referring to FIGS. 14 and 15, a second exemplary reconfigurable bushing 400 comprises a rectangular-shaped material having a substantially constant length L and thickness T. In exemplary form, the material may comprise a polymer, a metal, a metal alloy, a composite, or any other material that lends itself to reshaping and has at least a predetermined elasticity. By way of example, the reconfigurable bushing 400 comprises a metal alloy with a thickness T that allows a user to roll the bushing to change its width W. The more the bushing 400 is rolled over itself, the smaller its width W becomes (compare the smaller width W in FIG. 15 with the larger width W in FIG. 14). But when the affirmative pressure that has been applied to roll the bushing 400 is discontinued, the elasticity of the bushing causes the bushing to at least partially unroll and increase its width W.

This second exemplary reconfigurable bushing may be used in addition to or in lieu of the first reconfigurable bushing 220. When used as part of the first exemplary telescoping screw 100, the bushing 400 is able to be rolled so that its width W is smaller than the circular opening delineated by the flange 130 of the proximal sleeve 104 in order the distally pass through the flange (such as in FIG. 15). But after the bushing 400 passes distally beyond the flange 130, the bushing 400 at least partially unrolls to increase its width W so that the width is greater than the diameter of the circular opening delineated by the flange 130 in order to inhibit passing through the flange (such as in FIG. 14).

It should be noted that the second exemplary reconfigurable bushing 400 need not include a constant length or thickness. Rather, the length may be non-uniform and the bushing taking on a shape other than rectangular, whether or not the bushing is lying flat. Moreover, the thickness may be non-uniform so that the certain portions of the bushing retard deformation more or less than other portions.

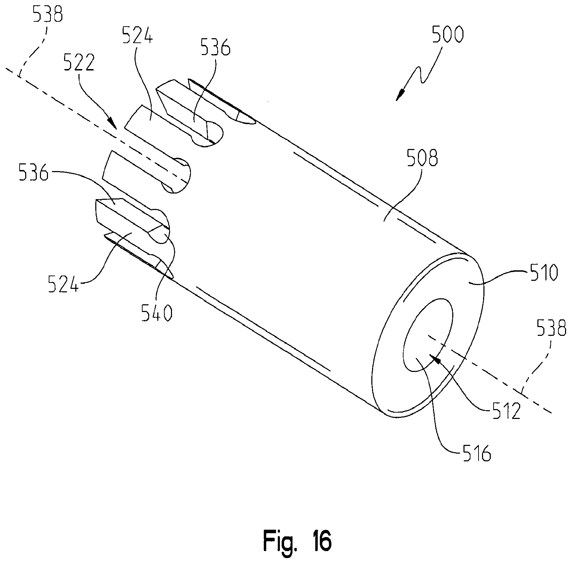

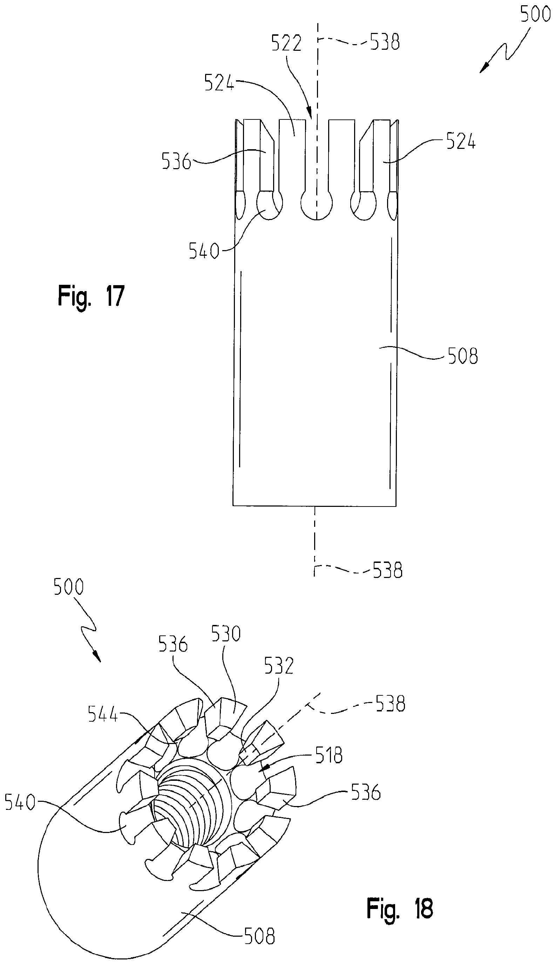

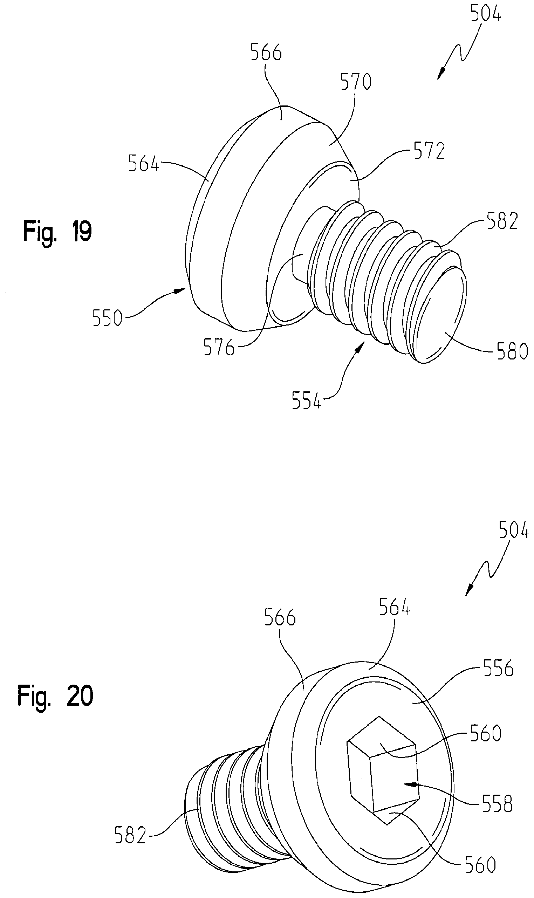

Referencing FIGS. 16-18, a third exemplary reconfigurable bushing 500 may be used in lieu of or in addition to the foregoing exemplary bushings 220, 400 as part of the exemplary telescoping screw 100. This reconfigurable bushing 500 is adapted to engage a positioning screw 504 (FIG. 19) in order to lock or fix the circumferential dimensions of the reconfigurable bushing. More specifically, insertion of the threaded end of the screw 504 into a proximal end of the bushing 500 may be operative to increase the circumferential cross-section of the bushing in order to wedge the bushing inside the cylindrical housing 114 of the proximal sleeve 102 or at the very least prohibit withdrawal of the bushing through the flange 130. In this fashion, by locating the bushing 500 within the inner circumferential wall 134 of the proximal sleeve 102, the maximum travel of the proximal sleeve with respect to the distal shaft 104 may be reduced.

The bushing 500, when not mounted to the positioning screw 504, includes a cylindrical shape having a substantially constant diameter along its longitudinal length. This cylindrical shape is typified by an outer circumferential surface 508 that is perpendicular to a substantially planar bottom surface 510. The bottom surface 510 is ring-shaped and partially delineates a through hole 512 that extends longitudinally along the length of the bushing. In exemplary form, the through hole 512 is bounded by an inner cylindrical surface 516 having a substantially constant diameter until terminating proximate a proximal end 518. The through hole 512 is axially centered along the longitudinal length of the bushing 500 and widens at its proximal end typified by cut-outs 522 that correspondingly form a series of circumferential projections 524.

In this exemplary embodiment, there are ten circumferential projections 524 that are interposed by ten cut-outs 522. However, the bushing may include more or fewer than ten projections 524. Likewise, while the projections 524 and cut-outs 522 are substantially uniform in shape, it is not required that this be the case.

Each projection 524 includes a tapered proximal surface 530 that declines from the outside perimeter joining the outer circumferential surface 508 to the inner perimeter that joins an inner circumferential surface 532. In this exemplary embodiment, the arcuate profile of the outer circumferential surface 508 is representative of a first ring and the arcuate profile of the inner circumferential surface 532 is representative of a second ring, where the first and second rings are concentric. Interposing the outer circumferential surface 508 and the inner circumferential surface 532 for each projection is a pair of planar surfaces 536 that lie upon a radian extending from a longitudinal axis 538. Joining adjacent planar surfaces 536 of adjacent projections 524 is a cylindrical surface 540 that partially defines a partial cylindrical cavity that extends perpendicularly out from the longitudinal axis 538. Each projection 524 includes a triangular plateau 544 extending radially toward the longitudinal axis 538, where each plateau is undercut as a result of the cylindrical surface 540. This undercut provides a cavity to receive at least a portion of the threads of the positioning screw 504 to facilitate longitudinal advancement of the positioning screw with respect to the bushing 500.

Referring to FIGS. 19 and 20, the positioning screw 504 includes a proximal head 550 from which a threaded shaft 554 extends perpendicularly. The proximal head 550 includes a top planar, circular surface 556 having a depression 558 formed therein. By way of example, the depression 558 is bounded by six vertical walls 560 to define a hexagonal cavity that may receive a hexagonal driver (now shown) to axially rotate the screw 504. Adjacent and surrounding the top surface 556 is a tapered surface 564 that links the top surface 556 with a circumferential surface 566 that is perpendicular to the top surface. Extending distally from the circumferential surface 566 is a frustroconical surface 570 that tapers from proximal to distal. Adjacent the frustroconical surface 570 is a bottom planar, circular surface 572 from which the threaded shaft 554 extends perpendicularly. In particular, the bottom surface 572 is parallel to the top planar surface 556 and spaced apart therefrom by the longitudinal length of the proximal head 550. Extending distally away from the proximal head 550 is the threaded shaft 554, which includes a cylindrical projection 576. This cylindrical projection 576 includes a substantially constant longitudinal diameter and planar bottom surface 580. A helical thread 582 circumscribes the cylindrical projection 576 and extends from proximate the bottom surface 572 until reaching the other bottom surface 580.

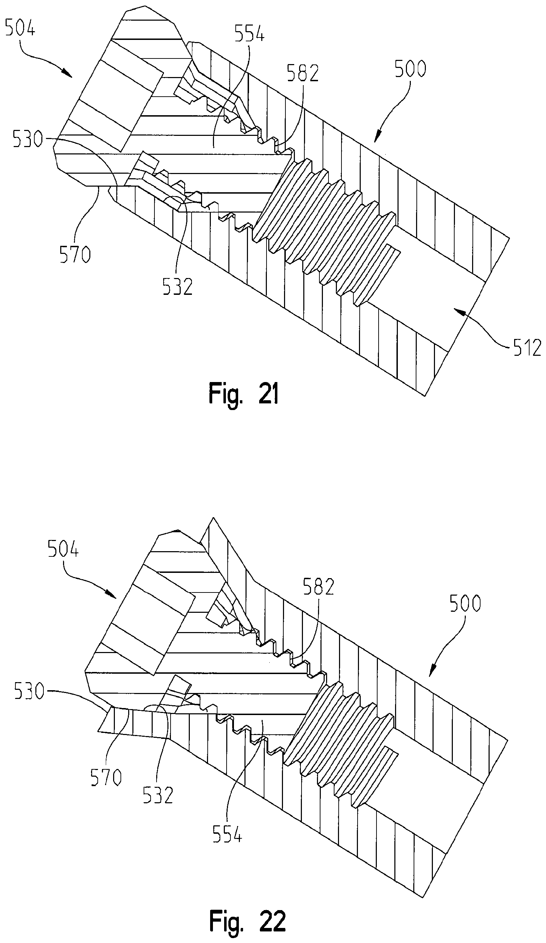

Referencing FIGS. 21 and 22, usage of the reconfigurable bushing 500 and the positioning screw 504 will now be described in the context of the proximal sleeve 102 and the distal shaft of the first exemplary telescoping screw (see FIGS. 1-5). Initially, the outside diameter of the bushing 500 is sized to allow passage into the proximal sleeve 102 via the circular opening delineated by the flange 130. Moreover, the outside diameter of the positioning screw 504 is also sized to allow passage into the proximal sleeve 102 via the circular opening delineated by the flange 130. While not required, it is envisioned that the bushing 500 and the positioning screw 504 will be mounted to one another as shown in FIG. 21 prior to insertion through the flange 130.

In order to mount the bushing 500 and positioning screw 504 to one another, the threaded shaft 554 is introduced into the through hole 512 of the bushing where the circumferential projections 524 are located. In this exemplary embodiment, the substantially constant diameter portion of the through hole 512 is slightly less than the outside diameter of the threaded shaft 554 so that the helical thread 582 engages the inner cylindrical surface 516. In this manner, rotation of the positioning screw 504 with respect to the bushing 500 causes the screw to longitudinally move proximally or distally with respect to the bushing. In this exemplary combination, clockwise rotation of the screw 504 causes the screw to move distally with respect to the bushing, while counterclockwise rotation of the screw causes the screw to move proximally with respect to the bushing. Eventually, clockwise rotation draws the threaded shaft 554 of the screw 504 deep enough so that at least one of the frustroconical surface 570 and the bottom surface 572 contacts the circumferential projections 524.

At this point, the screw 504 and bushing 500 may be moved through the proximal opening 122 of the proximal sleeve 102 and through the circular opening delineated by the flange 130. After passing the bushing 500 and the positioning screw 504 distally beyond the flange 130, while being retained within the interior of the proximal sleeve 102, a driver (not shown) may be rotated clockwise to draw the threaded shaft 554 of the screw 504 further into the bushing 500. By drawing the threaded shaft 554 of the screw 504 further into the bushing 500, the proximal head 550 is likewise drawn further into the bushing. In particular, the frustroconical nature of the circumferential surface 570 acts as a wedge to push against the tapered proximal surfaces 530 (and eventually against the inner circumferential surfaces 532) of the circumferential projections 524, thereby pushing the projections 524 outward away from the longitudinal axis 538. In so doing, the outer circumference of the bushing 500 increases and prohibits passage of the bushing and screw 504 proximally through the flange 130, while at the same time limiting travel of the distal shaft 102 with respect to the proximal sleeve 104.

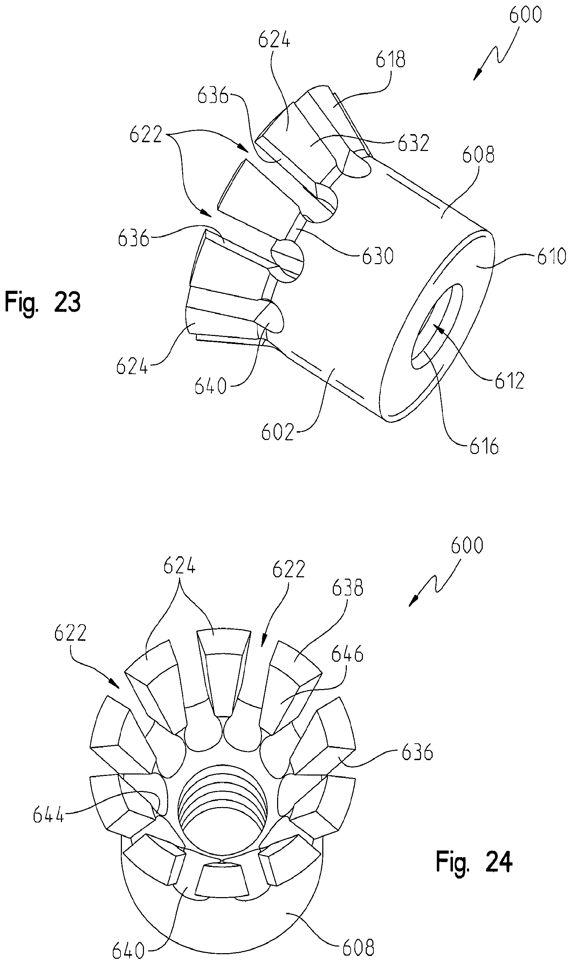

Referring to FIGS. 23 and 24, a fourth exemplary reconfigurable bushing 600 may be used in lieu of or in addition to the foregoing exemplary bushings 220, 400, 500 as part of the exemplary telescoping screw 100. This reconfigurable bushing 500 is adapted to engage a positioning screw 504 in order to lock or fix the circumferential dimensions of the reconfigurable bushing. Based upon the prior discussion of the positioning screw 504, a detailed discussion of this device has been omitted in furtherance of brevity.

Insertion of the threaded end of the screw 504 into a proximal end of the bushing 600 may be operative to increase the circumferential cross-section of the bushing in order to wedge the bushing inside the cylindrical housing 114 of the proximal sleeve 102 or at the very least prohibit withdrawal of the bushing through the flange 130. In this fashion, by locating the bushing 600 within the inner circumferential wall 134 of the proximal sleeve 102, the maximum travel of the proximal sleeve with respect to the distal shaft 104 may be reduced.

The bushing 600, when not mounted to the positioning screw 504, includes a cylindrical distal end 602 having a substantially constant diameter along its longitudinal length. This cylindrical distal end 602 is typified by an outer circumferential surface 608 that is perpendicular to a substantially planar bottom surface 610. The bottom surface 610 is ring-shaped and partially delineates a through hole 612 that extends longitudinally along the length of the bushing. In exemplary form, the through hole 612 is bounded by an inner cylindrical surface 616 having a substantially constant diameter until terminating proximate a proximal end 618 of the bushing 600. The through hole 612 is axially centered along the longitudinal length of the bushing 600 and widens at its proximal end typified by cut-outs 622 that correspondingly form a series of circumferential projections 624.

In this exemplary embodiment, there are ten circumferential projections 624 that are interposed by ten cut-outs 622. However, the bushing 600 may include more or fewer than ten projections 624. Likewise, while the projections 624 and cut-outs 622 are substantially uniform in shape, it is not required that this be the case.

Each projection 624 includes and arcuate exterior transition surface 630 that transitions from the cylindrical outer circumferential surface 608 to the outer circumferential surface 632. This outer circumferential surface 632 has a circumferential arc and longitudinal profile the mimics a frustroconical shape. Adjacent the outer circumferential surface 632 is a pair of planar lateral surfaces 636 that are also adjacent a proximal arcuate surface 638. Opposed lateral surfaces 632 for adjacent projections 624 are interposed by a cylindrical surface 640 that partially defines a partial cylindrical cavity that extends perpendicularly out from a longitudinal axis extending through the bushing 600. Each projection 624 includes a triangular plateau 644 extending radially toward the longitudinal axis, where each plateau is undercut as a result of the cylindrical surface 640. This undercut provides a cavity to receive at least a portion of the threads of the positioning screw 504 to facilitate longitudinal advancement of the positioning screw with respect to the bushing 600. Interconnecting the plateau 644 and the proximal surface 638 is an inner circumferential surface 646 having a circumferential arc and longitudinal profile the mimics a frustroconical shape.

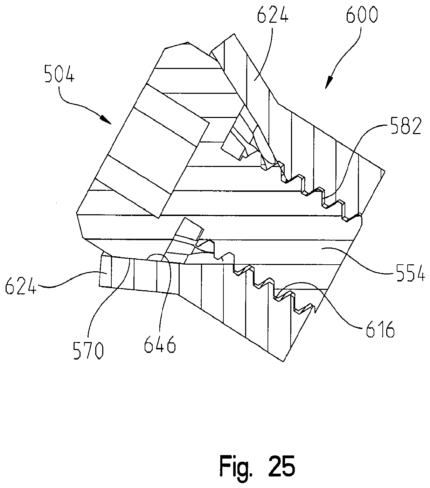

Referencing FIGS. 23-25, usage of the reconfigurable bushing 600 and the positioning screw 504 will now be described in the context of the proximal sleeve 102 and the distal shaft of the first exemplary telescoping screw (see FIGS. 1-5). Initially, the outside diameter of the bushing 600 is sized to allow passage into the proximal sleeve 102 via the circular opening delineated by the flange 130. Moreover, the outside diameter of the positioning screw 504 is also sized to allow passage into the proximal sleeve 102 via the circular opening delineated by the flange 130.

In order to mount the bushing 600 and positioning screw 504 to one another, the threaded shaft 554 is introduced into the through hole 612 of the bushing where the circumferential projections 624 are located. In this exemplary embodiment, the substantially constant diameter portion of the through hole 612 is slightly less than the outside diameter of the threaded shaft 554 so that the helical thread 582 engages the inner cylindrical surface 616. In this manner, rotation of the positioning screw 504 with respect to the bushing 600 causes the screw to longitudinally move proximally or distally with respect to the bushing. In this exemplary combination, clockwise rotation of the screw 504 causes the screw to move distally with respect to the bushing, while counterclockwise rotation of the screw causes the screw to move proximally with respect to the bushing. Eventually, clockwise rotation draws the threaded shaft 554 of the screw 504 deep enough so that the frustroconical surfaces 570 contact the circumferential projections 624.

Prior to mounting the screw 504 to the bushing 600, the screw 504 and bushing 600 are individually moved through the proximal opening 122 of the proximal sleeve 102 and through the circular opening delineated by the flange 130, with the distal surface 610 of the bushing passing though the flange first, followed by the screw. When passing the bushing 600 through the opening delineated by the flange 130, the circumferential projections 624 are deformed in order to decrease the outer circumference of the bushing at the proximal end in order to allow the bushing to completely pass beyond the flange. After passing beyond the flange 130, the circumferential projections 624 elastically reposition back to the position shown in FIGS. 23 and 24. After passing the bushing 600 and the positioning screw 504 distally beyond the flange 130, while being retained within the interior of the proximal sleeve 102, a driver (not shown) may engage the screw and rotate the threaded shaft 554 of the screw 504 to engage the bushing 500. By drawing the threaded shaft 554 of the screw 504 further into the bushing 600, the proximal head 550 is likewise drawn further into the bushing. In particular, the frustroconical nature of the circumferential surface 570 acts as a wedge to push against the inner circumferential surfaces 646 of the circumferential projections 624, thereby maintaining the position of the projections 624 outward away from the longitudinal axis. In so doing, the outer circumference of the bushing 600 prohibits passage of the bushing and screw 504 proximally through the flange 130, while at the same time limiting travel of the distal shaft 102 with respect to the proximal sleeve 104.

## Claims

1. A telescopic surgical screw (100) comprising:
a first screw section (104) including a distal threaded section and a proximal section (150);
a second screw section (102) including a proximal threaded head (110) and a distally extending cylindrical housing (114), wherein both the threaded head (110) and the cylindrical housing (114) are hollow representative of a longitudinal through hole that extends between a distal orifice (120) and a proximal orifice (122), said cylindrical housing (114) having a first width defined by an inner wall (134) and a second width defined by an internal flange (130), the second width being less than the first width; and,
a reconfigurable bushing (220, 400, 500, 600) configured to be received within the second screw section (102) to occupy a portion of the longitudinal through hole so as to limit travel of the first screw section (104) with respect to the second screw section, the bushing having an uncompressed width that is greater than the second width and further having a compressed width that is less than the second width, wherein the uncompressed width substantially prevents movement of the bushing from the inner wall (134) to the internal flange (130), wherein the bushing, while compressed, is slidable from the internal flange to the inner wall;
wherein the proximal section (150) of the first screw section (104) is slidable within the longitudinal through hole of the second screw section (102) between a first orientation where the first screw section and the second screw section define a first length and a second orientation where the first screw section and the second screw section define a second length.

2. The telescopic surgical screw of claim 1, wherein:
the distal section of the second screw section (102) includes an internal cavity sized to receive the proximal section (150) of the first screw section; and,
the distal section includes a distal collar (138) to inhibit complete disengagement between the first screw section and the second screw section.

3. The telescopic surgical screw of claim 1, wherein the reconfigurable bushing (220, 400, 500, 600) includes a discontinuous cylindrical profile along its longitudinal length.

4. The telescopic surgical screw of claim 1, wherein:
an inner diameter of the internal flange (130) is less than an outside diameter of the proximal section (150) of the first screw section;
at least a portion of the longitudinal through hole is bounded by a first inner circumferential surface (134) having a dimension greater than an exterior dimension of the proximal section of the first screw section;
at least a portion of the through hole is bounded by a second inner circumferential surface having a dimension less than the exterior dimension of the proximal section of the first screw section; and,
the first inner circumferential surface (134) interposes the second inner circumferential surface and the internal flange (130).

5. The telescopic surgical screw of claim 1, wherein the reconfigurable bushing (600) includes a distal section (602) having a uniform cylindrical profile and a proximal section having a tapered profile, the proximal section including a proximal cavity at least partially delineated by at least two fingers (624) that are deformable with respect to the distal section to change an outer circumference of the proximal section.

6. The telescopic surgical screw of claim 5, wherein:
the reconfigurable bushing includes a longitudinally extending through hole (612);
at least a portion of the longitudinally extending through hole is delineated by an inner circumferential surface of the distal section; and,
at least a portion of the longitudinally extending through hole is delineated by an inner surface of the at least two fingers.

7. The telescopic surgical screw of claim 6, wherein:
the at least two fingers comprise at least four fingers (624);
each of the four fingers are interposed by a cut-out (622); and,
each of the four fingers is circumferentially distributed at an end of the distal section.

8. The telescopic surgical screw of claim 1, wherein the reconfigurable bushing is fabricated from at least one of a polymer and a metal.

9. The telescopic surgical screw of claim 6, wherein:
the at least two fingers each include an inner surface (646) that faces an imaginary longitudinal axis extending through a center of the reconfigurable bushing; and,
the inner surfaces of the at least two fingers are at least one of threaded and textured.

10. The telescopic surgical screw of claim 1, wherein:
the reconfigurable bushing includes a distal section and a proximal section having a discontinuous wall at least partially delineating a proximal cavity; and,
an inner surface of the discontinuous wall at least partially delineating the proximal cavity is at least one of threaded and textured.

11. The telescopic surgical screw of claim 1, further comprising a positioning screw (504) configured to engage the reconfigurable bushing to retain a shape of the reconfigurable bushing to inhibit withdrawal from the internal cavity.

## Patentansprüche

1. Teleskopische chirurgische Schraube (100), umfassend:
einen ersten Schraubenabschnitt (104), der einen distalen Gewindeabschnitt und einen proximalen Abschnitt (150) umfasst;
einen zweiten Schraubenabschnitt (102), der einen proximalen Gewindekopf (110) und ein sich distal
erstreckendes zylindrisches Gehäuse (114) umfasst, wobei sowohl der Gewindekopf (110) als auch das zylindrische Gehäuse (114)
hohl sind, repräsentativ für ein Längsdurchgangsloch, das sich zwischen einer distalen Öffnung (120) und einer proximalen Öffnung (122) erstreckt, wobei das zylindrische Gehäuse (114) eine erste Breite, die durch eine Innenwand (134) definiert ist, und eine zweite Breite, die durch einen Innenflansch (130) definiert ist, aufweist, wobei die zweite Breite kleiner als die erste Breite ist; und
eine rekonfigurierbare Buchse (220, 400, 500, 600), die konfiguriert ist, um innerhalb des zweiten Schraubenabschnitts (102) aufgenommen zu werden, um einen Teil des Längsdurchgangslochs einzunehmen, um den Weg des ersten Schraubenabschnitts (104) in Bezug auf den zweiten Schraubenabschnitt zu begrenzen, wobei die Buchse eine nicht zusammengedrückte Breite aufweist, die größer als die zweite Breite ist, und ferner eine zusammengedrückte Breite aufweist, die kleiner als die zweite Breite ist, wobei die nicht zusammengedrückte Breite die Bewegung der Buchse von der Innenwand (134) zum Innenflansch (130) im Wesentlichen verhindert, wobei die Buchse, während sie zusammengedrückt ist, vom Innenflansch zur Innenwand verschiebbar ist;
wobei der proximale Abschnitt (150) des ersten Schraubenabschnitts (104) innerhalb des Längsdurchgangslochs des zweiten Schraubenabschnitts (102) zwischen einer ersten Ausrichtung, bei der der erste Schraubenabschnitt und der zweite Schraubenabschnitt eine erste Länge definieren, und einer zweiten Ausrichtung, bei der der erste Schraubenabschnitt und der zweite Schraubenabschnitt eine zweite Länge definieren, verschiebbar ist.

2. Teleskopische chirurgische Schraube nach Anspruch 1, wobei:
der distale Abschnitt des zweiten Schraubenabschnitts (102) einen inneren Hohlraum umfasst, der so bemessen ist, dass er den proximalen Abschnitt (150) des ersten Schraubenabschnitts aufnimmt; und
der distale Abschnitt einen distalen Kragen (138) umfasst, um ein vollständiges Lösen zwischen dem ersten Schraubenabschnitt und dem zweiten Schraubenabschnitt zu verhindern.

3. Teleskopische chirurgische Schraube nach Anspruch 1, wobei die rekonfigurierbare Buchse (220, 400, 500, 600) entlang ihrer Längslänge ein diskontinuierliches zylindrisches Profil umfasst.

4. Teleskopische chirurgische Schraube nach Anspruch 1, wobei:
ein Innendurchmesser des Innenflansches (130) kleiner als ein Außendurchmesser des proximalen Abschnitts (150) des ersten Schraubenabschnitts ist;
mindestens ein Teil des Längsdurchgangslochs ist durch eine erste innere Umfangsfläche (134) begrenzt ist, die eine Abmessung aufweist, die größer als eine Außenabmessung des proximalen Abschnitts des ersten Schraubenabschnitts ist;
mindestens ein Teil des Durchgangslochs durch eine zweite innere Umfangsfläche begrenzt ist, die eine Abmessung aufweist, die kleiner als die Außenabmessung des proximalen Abschnitts des ersten Schraubenabschnitts ist; und
die erste innere Umfangsfläche (134) zwischen der zweiten inneren Umfangsfläche und dem Innenflansch (130) liegt.

5. Teleskopische chirurgische Schraube nach Anspruch 1, wobei die rekonfigurierbare Buchse (600) einen distalen Abschnitt (602) umfasst, der ein gleichmäßiges zylindrisches Profil und einen proximalen Abschnitt aufweist, der ein
sich verjüngendes Profil aufweist, wobei der proximale Abschnitt einen proximalen Hohlraum umfasst, der zumindest teilweise durch mindestens zwei Finger (624) abgegrenzt ist, die in Bezug auf den distalen Abschnitt verformbar sind, um einen Außenumfang des proximalen Abschnitts zu verändern.

6. Teleskopische chirurgische Schraube nach Anspruch 5, wobei:
die rekonfigurierbare Buchse ein in Längsrichtung verlaufendes Durchgangsloch (612) umfasst; mindestens ein Teil des sich in Längsrichtung erstreckenden Durchgangslochs durch eine innere Umfangsfläche des distalen Abschnitts abgegrenzt ist; und
mindestens ein Teil des sich in Längsrichtung erstreckenden Durchgangslochs durch eine Innenfläche der mindestens zwei Finger abgegrenzt ist.

7. Teleskopische chirurgische Schraube nach Anspruch 6, wobei:
die mindestens zwei Finger mindestens vier Finger (624) umfassen; jeder der vier Finger durch einen Ausschnitt (622) dazwischen eingefügt ist; und,
jeder der vier Finger in Umfangsrichtung an einem Ende des distalen Abschnitts verteilt ist.

8. Teleskopische chirurgische Schraube nach Anspruch 1, wobei die rekonfigurierbare Buchse aus mindestens einem von einem Polymer und einem Metall hergestellt ist.

9. Teleskopische chirurgische Schraube nach Anspruch 6, wobei:
die mindestens zwei Finger jeweils eine Innenfläche (646) umfassen, die einer imaginären Längsachse zugewandt ist, die sich durch eine Mitte der rekonfigurierbaren Buchse erstreckt; und
die Innenflächen der mindestens zwei Finger mindestens eines von mit Gewinde versehen und strukturiert sind.

10. Teleskopische chirurgische Schraube nach Anspruch 1, wobei:
die rekonfigurierbare Buchse einen distalen Abschnitt und einen proximalen Abschnitt, der eine diskontinuierliche Wand aufweist, die einen proximalen Hohlraum zumindest teilweise abgrenzt, umfasst; und
eine innere Oberfläche der diskontinuierlichen Wand, die den proximalen Hohlraum zumindest teilweise abgrenzt, mindestens eines von mit Gewinde versehen und strukturiert ist.

11. Teleskopische chirurgische Schraube nach Anspruch 1, ferner umfassend eine Positionierungsschraube (504), die konfiguriert ist, um in die rekonfigurierbare Buchse einzugreifen, um eine Form der rekonfigurierbaren Buchse beizubehalten, um ein Herausziehen aus dem inneren Hohlraum zu verhindern.

## Revendications

1. Vis chirurgicale télescopique (100) comprenant :
une première section de vis (104) comprenant une section distale filetée et une section proximale (150) ;
une seconde section de vis (102) comprenant une tête proximale filetée (110) et un boîtier cylindrique s'étendant de manière distale (114), aussi bien la tête filetée (110) que le boîtier cylindrique (114) étant creux représentatifs d'un trou traversant longitudinal qui s'étend entre un orifice distal (120) et un orifice proximal (122), ledit boîtier cylindrique (114) présentant une première largeur définie par une paroi interne (134) et une seconde largeur définie par un rebord interne (130), la seconde largeur étant inférieure à la première largeur ; et,
une douille reconfigurable (220, 400, 500, 600) conçue pour être reçue dans la seconde section de vis (102) afin d'occuper une partie du trou traversant longitudinal de manière à limiter le déplacement de la première section de vis (104) par rapport à la seconde section de vis, la douille présentant une largeur non comprimée qui est supérieure à la seconde largeur et présentant en outre une largeur comprimée qui est inférieure à la seconde largeur, ladite largeur non comprimée empêchant sensiblement le déplacement de la douille de la paroi interne (134) vers le rebord interne (130), ladite douille, lorsqu'elle est comprimée, pouvant coulisser du rebord interne vers la paroi interne ;
ladite section proximale (150) de la première section de vis (104) pouvant coulisser à l'intérieur du trou traversant longitudinal de la seconde section de vis (102) entre une première orientation dans laquelle la première section de vis et la seconde section de vis définissent une première longueur et une seconde orientation dans laquelle la première section de vis et la seconde section de vis définissent une seconde longueur.

2. Vis chirurgicale télescopique selon la revendication 1 :
ladite section distale de la seconde section de vis (102) comprenant une cavité interne dimensionnée pour recevoir la section proximale (150) de la première section de vis ; et,
ladite section distale comprenant un collier distal (138) pour empêcher un désengagement complet entre la première section de vis et la seconde section de vis.

3. Vis chirurgicale télescopique selon la revendication 1, ladite douille reconfigurable (220, 400, 500, 600) comprenant un profil cylindrique discontinu sur sa longueur longitudinale.

4. Vis chirurgicale télescopique selon la revendication 1 :
un diamètre interne du rebord interne (130) étant inférieur à un diamètre externe de la section proximale (150) de la première section de vis ;
au moins une partie du trou traversant longitudinal étant délimitée par une première surface circonférentielle interne (134) présentant une dimension supérieure à une dimension extérieure de la section proximale de la première section de vis ;
au moins une partie du trou traversant étant délimitée par une seconde surface circonférentielle interne présentant une dimension inférieure à la dimension extérieure de la section proximale de la première section de vis ; et,
la première surface circonférentielle interne (134) étant intercalée entre la seconde surface circonférentielle interne et le rebord interne (130).

5. Vis chirurgicale télescopique selon la revendication 1, ladite douille reconfigurable (600) comprenant une section distale (602) qui présente un profil cylindrique uniforme et une section proximale qui présente un profil effilé, la section proximale comprenant une cavité proximale au moins partiellement délimitée par au moins deux pattes (624) qui sont déformables par rapport à la section distale pour modifier une circonférence externe de la section proximale.

6. Vis chirurgicale télescopique selon la revendication 5 :
ladite douille reconfigurable comprenant un trou traversant s'étendant longitudinalement (612) ;
au moins une partie du trou traversant s'étendant longitudinalement étant délimitée par une surface circonférentielle interne de la section distale ; et,
au moins une partie du trou traversant s'étendant longitudinalement étant délimitée par une surface interne desdites au moins deux pattes.

7. Vis chirurgicale télescopique selon la revendication 6 :
lesdites au moins deux pattes comprenant au moins quatre pattes (624) ;
une découpe (622) étant intercalée entre chacune des quatre pattes ; et,
chacune des quatre pattes étant répartie circonférentiellement au niveau d'une extrémité de la section distale.

8. Vis chirurgicale télescopique selon la revendication 1, ladite douille reconfigurable étant fabriquée à partir d'au moins l'un d'un polymère et d'un métal.

9. Vis chirurgicale télescopique selon la revendication 6 :
lesdites au moins deux pattes comprenant chacune une surface interne (646) qui fait face à un axe longitudinal imaginaire s'étendant à travers un centre de la douille reconfigurable ; et,
lesdites surfaces internes desdits au moins deux pattes étant au moins l'une parmi une filetée et une texturée.

10. Vis chirurgicale télescopique selon la revendication 1 :
ladite douille reconfigurable comprenant une section distale et une section proximale présentant une paroi discontinue qui délimite au moins partiellement une cavité proximale ; et,
une surface interne de la paroi discontinue qui délimite au moins partiellement la cavité proximale étant au moins l'une parmi une filetée et une texturée.

11. Vis chirurgicale télescopique selon la revendication 1, comprenant en outre une vis de positionnement (504) conçue pour venir en prise avec la douille reconfigurable pour maintenir une forme de la douille reconfigurable afin d'empêcher le retrait de la cavité interne.
